# EUROPÄISCHE PATENTANMELDUNG

(11) **EP 4 702 938 A1**
(43) Veröffentlichungstag der Anmeldung: **04.03.2026**
(21) Anmeldenummer: 25197998.5
(22) Anmeldetag: 26.08.2025
(51) Int. Cl.: A61B 17/70

(54) **MEDIZINISCHE SCHRAUBE UND MEDIZINISCHE SCHRAUBENVORRICHTUNG**

(30) Priorität: 27.08.2024 DE 102024124421
(71) Anmelder: Aesculap AG, 78532 Tuttlingen (DE)
(72) Erfinder: PEUKERT, Andrea, 78532 Tuttlingen (DE); MAUCH, Simone, 88605 Messkirch (DE); MARX, Irene, 78647 Trossingen (DE); RICCI, Denis, 78573 Wurmlingen (DE)
(74) Vertreter: Winter, Brandl - Partnerschaft mbB

(57) **Zusammenfassung**

Die vorliegende Offenbarung betrifft eine medizinische Schraube (1), ausgebildet und vorgesehen zum wahlweisen Einschrauben in das Sakrum (2) und das Ilium (3) eines Patienten, mit einem Gewindeschaft (4), dessen proximaler Endabschnitt (4.pE) zu einem Kugelkopf (5) ausgebildet ist, einer hülsenförmigen Tulpe (6), die an ihrem distalen axialen Endabschnitt (6.dE) bezüglich des Gewindeschafts (4) schwenk- und drehbar am Kugelkopf (5) gehalten ist und die eine, an der proximalen Stirnseite (6.pS) der Tulpe (6) offene Querausnehmung (7) aufweist, die zur Queraufnahme eines Stabilisierungsstabs (8) vorgesehen ist, wobei die Querausnehmung (7) im proximalen axialen Endabschnitt (6.pE) der Tulpe (6) mit einem Innengewinde (9) ausgebildet ist und einer Madenschraube (10), die in das Innengewinde (9) eingedreht oder eindrehbar ist sowie dafür vorgesehen ist, einen in die Querausnehmung (7) eingesetzten Stabilisierungsstab (8) direkt oder indirekt gegen den Kugelkopf (5) zu drücken und die Tulpe (6) am Kugelkopf (5) in einer auswählbaren Relativausrichtung zu fixieren, gekennzeichnet durch eine, an oder von der distalen Stirnseite (6.dS) der Tulpe (6) ausgebildete, den Schwenkwinkel (α) der Tulpe (6) bezüglich des Gewindeschafts (4) vergrößernde Abschrägung (11), deren, in Umfangsrichtung (u) gesehene, radiale Mittelachse (11.r) in einer Tulpen-Radialrichtung zwischen einer Querausnehmungsachse (7.A) und einer hierzu um 90° gedrehten Tulpenquerachse (6.A) ausgerichtet ist. Ferner betrifft die vorliegende Offenbarung ein medizinische Schraubenvorrichtung (13), gekennzeichnet durch wenigstens zwei solche medizinischen Schrauben (1).

## Beschreibung

Die vorliegende Offenbarung betrifft eine medizinische Schraube gemäß dem Oberbegriff des Patentanspruchs 1, 2 und 3, sowie eine medizinische Schraubenvorrichtung bzw. Schraubsystem gemäß dem Oberbegriff des Patentanspruchs 8.

### Hintergrund der Offenbarung

Es gibt medizinische Schrauben, die ausgebildet und vorgesehen sind, in das Sakrum eines Patienten eingeschraubt zu werden. Es gibt weitere medizinische Schrauben, die ausgebildet und vorgesehen sind, in das Ilium eines Patienten eingeschraubt zu werden. Beide Schraubentypen werden häufig im Rahmen eines Schraubsystems gemeinsam verwendet, um die untere Wirbelsäule eines Patienten im Beckenbereich zu stabilisieren. Solche Schrauben weisen grundsätzlich einen Gewindeschaft auf, dessen proximaler Endabschnitt zu einem Kugelkopf ausgebildet ist. Die Schrauben weisen weiterhin eine hülsenförmige Tulpe auf, die an ihrem distalen axialen Endabschnitt bezüglich des Gewindeschafts schwenk- und drehbar am Kugelkopf gehalten ist und die eine, an der proximalen Stirnseite der Tulpe offene Querausnehmung aufweist, die zur Queraufnahme eines Stabilisierungsstabs vorgesehen ist, wobei die Querausnehmung im proximalen axialen Endabschnitt der Tulpe mit einem Innengewinde ausgebildet ist. Die Schrauben weisen schließlich eine Madenschraube auf, die in das Innengewinde eingedreht oder eindrehbar ist sowie dafür vorgesehen ist, einen in die Querausnehmung quer zur Tulpe eingesetzten Stabilisierungsstab direkt oder indirekt gegen den Kugelkopf zu drücken und so die Tulpe am Kugelkopf in einer auswählbaren Relativausrichtung zu fixieren und gleichzeitig den Stabilisierungsstab in der Tulpe zu verspannen.

Derartige Schrauben sind insbesondere Teil einer übergeordneten medizinischen Schraubenvorrichtung (Schraubensystem). Eine solche medizinische Schraubenvorrichtung ist, wie vorstehend bereits angedeutet, zum Einschrauben in das Sakrum und das Ilium eines Patienten sowie zum Stabilisieren der Verbindung zwischen dem Sakrum und dem Ilium ausgebildet und vorgesehen.

### Stand der Technik

Es sind Schrauben bekannt, welche zur Aufnahme von, von cranial nach caudal verlaufenden Stabilisierungsstäben dienen und welche eine, an oder von der distalen Stirnseite der Tulpe ausgebildete, den Schwenkwinkel der Tulpe bezüglich des Gewindeschafts vergrößernde Abschrägung aufweisen, deren, in Umfangsrichtung gesehene, radiale Mittelachse in einer Tulpen-Radialrichtung mit 90° zu der Querausnehmungsachse ausgerichtet ist. D.h., derartige Tulpen können quer zur Verbindungsstab-Längsrichtung weiter am Kugelkopf geschwenkt werden als in Verbindungsstab-Längsrichtung. Solche Schrauben können auf beiden Seiten der Wirbelsäule eingesetzt werden und sind dazu ausgebildet und vorgesehen, durch das Sakrum hindurch und in das Ilium eingeschraubt zu werden. Man spricht dabei auch von einer sakroiliakalen Verschraubung. Solche Schrauben ermöglichen aber keine direkte Anbindung an in medial-lateraler Richtung verlaufende Stäbe.

Es sind weiterhin Schrauben zur Aufnahme von, von medial nach lateral verlaufenden Stäben, nämlich insbesondere sogenannten Seitenversatz-Ausgleichsstäben, bekannt, welche eine, an oder von der distalen Stirnseite der Tulpe ausgebildete, den Schwenkwinkel der Tulpe bezüglich des Gewindeschafts vergrößernde Abschrägung aufweisen, deren, in Umfangsrichtung gesehene, radiale Mittelachse in einer Tulpen-Radialrichtung parallel zu der Querausnehmungsachse ausgerichtet ist. D.h., derartige Tulpen können in Verbindungsstab-Längsrichtung weiter am Kugelkopf geschwenkt werden als quer zur Verbindungsstab-Längsrichtung. Auch diese Schrauben können auf beiden Seiten der Wirbelsäule eingesetzt werden und sind weiterhin dazu ausgebildet und vorgesehen, in das Ilium eingeschraubt zu werden. Man spricht dabei auch von einer Beckenverschraubung. Solche Schrauben ermöglichen aber keine direkte Anbindung an in cranial-caudaler Richtung verlaufende Stabilisierungsstäbe, da diese Ilium-Schrauben lateraler sitzen. Für eine solche Verbindung ist dann ein Lateral Offset Connector (LOC), ein Connector mit integriertem Seitenversatz-Ausgleichsstab, oder ein Sakral-Connector, der sowohl einen medial-lateralen als auch einen cranialcaudalen Stab aufnehmen kann, notwendig.

Das Segment sakroiliakaler Verschraubung und Beckenverschraubung ist aber ein relativ kleines Marktsegment, wo es sich nicht lohnt zwei verschiedene Schraubentypen anzubieten, so dass man sich in der Praxis für eine der beiden Varianten dieser Versorgungen entscheiden muss.

### Kurzbeschreibung der Offenbarung

Der vorliegenden Offenbarung liegt nun die Aufgabe zugrunde, eine medizinische Schraube und eine medizinische Schraubenvorrichtung bereitzustellen, welche die Probleme des Stands der Technik reduzieren oder beseitigen. Insbesondere soll die Schraube sowohl für die Beckenverschraubung und die sakroiliakale Verschraubung also für die Aufnahme von von cranial nach caudal verlaufenden Stabilisierungsstäben und von von medial nach lateral verlaufenden Stäben geeignet sein.

Diese Aufgabe wird zunächst durch eine medizinische Schraube gemäß Anspruch 1 gelöst. Weitere vorteilhafte Ausführungsformen sind Gegenstand der Unteransprüche.

Genauer wird die Aufgabe gelöst durch eine, an oder von der distalen Stirnseite der Tulpe ausgebildete, den Schwenkwinkel der Tulpe bezüglich des Gewindeschafts vergrößernde Abschrägung, deren, in Umfangsrichtung gesehene, radiale Mittelachse in einer Tulpen-Radialrichtung zwischen einer Querausnehmungsachse und einer hierzu um 90° gedrehten Tulpenquerachse ausgerichtet ist.

Durch die Ausrichtung der radialen Mittelachse zwischen der Querausnehmungsachse und der Tulpenquerachse ist ermöglicht, dass die Tulpe gegenüber dem Gewindeschaft sowohl parallel zur Tulpenquerachse wie auch parallel zur Querausnehmungsachse ausreichend stark verschwenkbar ist, um sowohl eine Verbindung mit einem von cranial nach caudal verlaufenden Stabilisierungsstab wie auch mit einem von medial nach lateral verlaufenden Stab zu ermöglichen. Es ist mit dieser einen Schraube dann sowohl die eingangs beschriebene Beckenverschraubung wie auch die sakroiliakale Verschraubung möglich. Somit können baugleiche Schrauben für mehr Patienten genutzt werden, was sich positiv auf die Herstellkosten dieser Schrauben auswirkt.

Die Aufgabe wird ferner durch eine medizinische Schraube gemäß Anspruch 2 gelöst.

Genauer wird die Aufgabe dann gelöst durch zwei an oder von der distalen Stirnseite der Tulpe ausgebildete, den Schwenkwinkel der Tulpe bezüglich des Gewindeschafts jeweils vergrößernde Aussparungen, deren, in Umfangsrichtung gesehene, radiale Mittelachsen in einer Tulpen-Radialrichtung auf einer Querausnehmungsachse und einer hierzu um 90° gedrehten Tulpenquerachse platziert sind.

Auch durch diese beiden Aussparungen ist ermöglicht, dass die Tulpe gegenüber dem Gewindeschaft sowohl parallel zur Tulpenquerachse wie auch parallel zur Querausnehmungsachse ausreichend stark verschwenkbar ist, um sowohl eine Verbindung mit einem von cranial nach caudal verlaufenden Stabilisierungsstab wie auch mit einem von medial nach lateral verlaufenden Stab zu ermöglichen. Es ist auch mit dieser einen Schraube dann sowohl die eingangs beschriebene Beckenverschraubung wie auch die sakroiliakale Verschraubung möglich. Somit können baugleiche Schrauben für mehr Patienten genutzt werden, was sich positiv auf die Herstellkosten dieser Schrauben auswirkt.

Die Aufgabe wird ferner durch eine medizinische Schraube gemäß Anspruch 3 gelöst.

Genauer wird die Aufgabe dann gelöst durch eine zumindest zweiteilige Tulpe mit einem die Querausnehmung aufweisenden Stab-Bereich und mit einem den Kugelkopf aufnehmenden Gelenk-Bereich, welche vorzugsweise senkrecht zur Querausnehmungsachse verdrehbar zueinander sind, um eine in Umfangsrichtung gesehene, radiale Mittelachse, einer an oder von der distalen Stirnseite der Tulpe ausgebildeten, den Schwenkwinkel der Tulpe bezüglich des Gewindeschafts vergrößernden Abschrägung oder Aussparung relativ zur Querausnehmungsachse einzustellen.

Auch durch die Verdrehbarkeit des Stab-Bereichs zum Gelenk-Bereich ist ermöglicht, dass die Tulpe gegenüber dem Gewindeschaft sowohl parallel zur Tulpenquerachse wie auch parallel zur Querausnehmungsachse ausreichend stark verschwenkbar ist, um sowohl eine Verbindung mit einem von cranial nach caudal verlaufenden Stabilisierungsstab wie auch mit einem von medial nach lateral verlaufenden Stab zu ermöglichen. Dazu muss dann nur vorher die entsprechende Verdrehung mit der gewünschten Einstellung der radialen Mittelachse stattfinden. Es ist auch mit dieser einen Schraube dann sowohl die eingangs beschriebene Beckenverschraubung wie auch die sakroiliakale Verschraubung möglich. Somit können baugleiche Schrauben für mehr Patienten genutzt werden, was sich positiv auf die Herstellkosten dieser Schrauben auswirkt.

Es kann vorteilhaft sein, wenn die radiale Mittelachse der Abschrägung um 40° bis 50°, bevorzugt um 43° bis 47°, insbesondere um 45°, zur Querausnehmungsachse gedreht ist. Dann ist die radiale Mittelachse im Wesentlichen mittig zwischen der Querausnehmungsachse und der Tulpenquerachse ausgerichtet. So sind auf einfache Weise gleich große Verschwenkbarkeiten der Tulpe gegenüber dem Gewindeschaft parallel zur Tulpenquerachse wie auch parallel zur Querausnehmungsachse realisierbar.

Vorteilhafterweise ist der Neigungswinkel der Abschrägung bezüglich der Mittelachse des Innengewindes so gewählt, dass ein möglicher Schwenkwinkel der Tulpe in Richtung der Querausnehmungsachse und der 90° dazu gedrehten Tulpenquerachse jeweils Werte von 30° bis 46°, vorzugsweise von 35° bis 41°, insbesondere von 38°, aufweist. Der mögliche Schwenkwinkel der Tulpe ist zwischen einer Mittestellung der Tulpe und einer maximal zur Abschrägung hin verschwenkten Tulpe ausgebildet. In der Mittestellung der Tulpe ist die Mittelachse des Gewindeschafts senkrecht zur mittels der Querausnehmungsachse und der Tulpenquerachse aufgespannten Ebene ausgerichtet. In der Mittestellung der Tulpe sind die Mittelachse des Gewindeschafts und die Mittelachse des Innengewindes vorzugsweise deckungsgleich oder liegen vorzugsweise zumindest parallel zueinander. Durch Realisierung derartig großer möglicher Schwenkwinkel sind ein Großteil der sinnvollen Beckenverschraubungen wie auch sakroiliakalen Verschraubungen ermöglicht.

Weiter bevorzugt ist die Aussparungstiefe beider Aussparungen so gewählt, dass ein möglicher Schwenkwinkel der Tulpe in Richtung der Querausnehmungsachse und der 90° dazu gedrehten Tulpenquerachse jeweils Werte von 30° bis 46°, vorzugsweise von 35° bis 41°, insbesondere von 38°, aufweist. Die möglichen Schwenkwinkel sind dabei wie oben beschrieben bestimmbar, wobei sich hier die radiale Mittelachse aber nicht auf die Abschrägung bezieht, sondern wobei zwei radiale Mittelachsen vorgesehen sind, welche sich auf die beiden Aussparungen beziehen. Auch durch diese Realisierung derartiger möglicher Schwenkwinkel sind ein Großteil der sinnvollen Beckenverschraubungen wie auch sakroiliakalen Verschraubungen ermöglicht.

Nach einer vorteilhaften Ausführungsform der medizinischen Schraube ist mittels der Aussparung oder Abschrägung eine ebene Kontakt-Fläche zwischen der Tulpe und dem Schraubenschaft gebildet. Die Tulpe ist gegenüber dem Gewindeschaft verschwenkbar, bis ein Kontakt zwischen der Tulpe und dem Schraubenschaft eben insbesondere im Bereich der Kontakt-Fläche erzeugt ist. Der Kontakt ist insbesondere in einem dem Schraubenschaft zugewandten Randbereich der Kontakt-Fläche erzeugbar. Der Schraubenschaft ist ein Bereich der Schraube ohne Gewinde, welcher an den Kugelkopf angrenzt. Die Tulpe ist gegenüber dem Gewindeschaft auch in anderen Richtungen als zur Aussparung oder Abschrägung hin verschwenkbar, aber dann eben nur um kleinere Schwenkwinkel.

Die Aufgabe wird ferner durch eine medizinische Schraubenvorrichtung (Schraubensystem) gemäß Anspruch 8 gelöst.

Genauer wird die Aufgabe dann gelöst durch eine medizinische Schraubenvorrichtung mit wenigstens zwei im Vorhinein beschriebenen, medizinischen Schrauben, einem Stabilisierungsstab zur starren Kopplung des Sakrums mit einem unmittelbar sich an diesen anschließenden Wirbelknochen, sowie einem Seitenversatz-Ausgleichsstab zur starren Kopplung des Sakrums mit dem Ilium.

Eine der beiden Schrauben ist dabei vorzugsweise mit dem Stabilisierungsstab, die andere vorzugsweise mit dem Seitenversatz-Ausgleichsstab verbunden. Für diese medizinische Schraubenvorrichtung können dann vorzugsweise zwei baugleiche medizinische Schrauben genutzt werden, auch wenn die eine mit dem Stabilisierungsstab und die andere mit dem Seitenversatz-Ausgleichsstab verbunden ist, was sich positiv auf die Kosten der medizinischen Schraubenvorrichtung auswirkt.

### Kurzbeschreibung der Figuren

Im Nachfolgenden werden bevorzugte Ausführungsformen unter Bezugnahme auf die anliegenden Figuren näher dargestellt.
Fig. 1a zeigt schematisch eine erste Ausführungsform einer medizinischen Schraube in einer Seitenansicht in Richtung einer Querausnehmungsachse.
Fig. 1b zeigt schematisch die erste Ausführungsform der medizinischen Schraube in einer Draufsicht auf eine proximale Stirnseite einer Tulpe der medizinischen Schraube.
Fig. 1c zeigt schematisch die erste Ausführungsform der medizinischen Schraube in einer Schnittansicht entlang der Linie A-A aus Fig.1b.
Fig. 1d zeigt schematisch die erste Ausführungsform der medizinischen Schraube in einer Schnittansicht entlang der Linie B-B aus Fig.1b.
Fig. 2a zeigt stark schematisch die erste Ausführungsform der medizinischen Schraube in einer Draufsicht.
Fig. 2b zeigt stark schematisch eine zweite Ausführungsform der medizinischen Schraube in einer Draufsicht.
Fig. 3a zeigt schematisch eine dritte Ausführungsform einer medizinischen Schraube in einer Schnittansicht entlang der Mittelachse eines Innengewindes der Tulpe und der Mittelachse eines Gewindeschafts der medizinischen Schraube mit einem realisierten maximalen Schwenkwinkel der Tulpe.
Fig. 3b zeigt schematisch die dritte Ausführungsform der medizinischen Schraube gemäß Fig.3a aber mit einem realisierten anderen Schwenkwinkel der Tulpe.
Fig.4a bis 4d zeigen verschiedene Anwendungsmöglichkeiten der medizinischen Schraube, insbesondere in Fig.4b eine erfindungsgemäße medizinische Schraubenvorrichtung.

### Beschreibung bevorzugter Ausführungsbeispiele

Die medizinische Schraube 1, ausgebildet und vorgesehen zum wahlweisen Einschrauben in das Sakrum 2 und das Ilium 3 eines Patienten, hat u.a. einen Gewindeschaft 4, dessen proximaler Endabschnitt 4.pE zu einem Kugelkopf 5 ausgebildet ist. Die medizinische Schraube 1 hat weiterhin eine hülsenförmige Tulpe 6, die an ihrem distalen axialen Endabschnitt 6.dE bezüglich des Gewindeschafts 4 schwenk- und drehbar am Kugelkopf 5 gehalten ist und die eine, an der proximalen Stirnseite 6.pS der Tulpe 6 offene Querausnehmung 7 aufweist, die zur Queraufnahme eines Stabilisierungsstabs 8 vorgesehen ist, wobei die Querausnehmung 7 im proximalen axialen Endabschnitt 6.pE der Tulpe 6 mit einem Innengewinde 9 ausgebildet ist. Die medizinische Schraube 1 hat weiterhin eine Madenschraube 10, die in das Innengewinde 9 eingedreht oder eindrehbar ist sowie dafür vorgesehen ist, einen in die Querausnehmung 7 eingesetzten Stabilisierungsstab 8 direkt oder indirekt gegen den Kugelkopf 5 zu drücken und die Tulpe 6 am Kugelkopf 5 in einer auswählbaren Relativausrichtung zu fixieren.

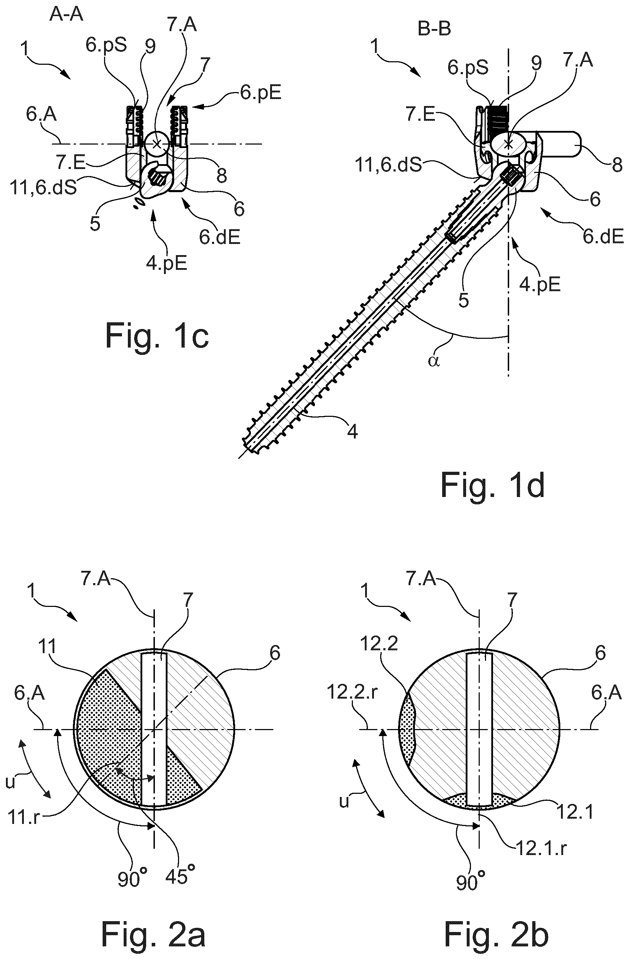

Mit indirekt ist hier gemeint, dass ein Einsatz 7.E gemäß Fig.1c und Fig.1d vorgesehen sein kann, welcher zwischen dem Stabilisierungsstab 8 und dem Kugelkopf 5 anordenbar ist und welcher von dem Stabilisierungsstab 8 gegen den Kugelkopf 5 drückbar ist, um das Bewegen des Kugelkopfes 5 gegenüber der Tulpe 6, insbesondere durch Reibschluss, zu blockieren. Der Einsatz 7.E wird auch als Insert bezeichnet. Der Einsatz 7.E ist durch den Stabilisierungsstab 8 verformbar und somit ist der Kugelkopf 5 gegenüber der Tulpe 6 mit dem Einsatz 7.E einklemmbar. Der Kugelkopf 5 ist vorzugsweise rotationssymmetrisch ausgeführt. Insbesondere ist der gesamte Gewindeschaft 4 bis auf die Gewindegänge rotationssymmetrisch ausgeführt. Insbesondere ist ein Zwischenbereich zwischen dem Kugelkopf 5 und den Gewindegängen, nämlich ein Schraubenschaft, rotationssymmetrisch ausgeführt.

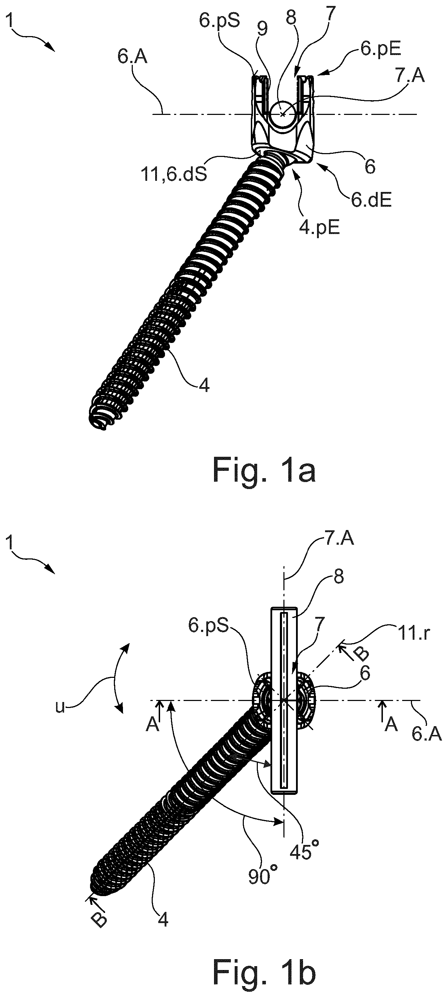

Gemäß der ersten Ausführungsform der medizinischen Schraube 1 aus Fig.1a bis Fig.1d und Fig.2a weist die medizinische Schraube 1 eine, an oder von der distalen Stirnseite 6.dS der Tulpe 6 ausgebildete, den Schwenkwinkel α der Tulpe 6 bezüglich des Gewindeschafts 4 vergrößernde Abschrägung 11 auf, deren, in Umfangsrichtung u gesehene, radiale Mittelachse 11.r in einer Tulpen-Radialrichtung zwischen einer Querausnehmungsachse 7.A und einer hierzu um 90° gedrehten Tulpenquerachse 6.A ausgerichtet ist.

Die Tulpe 6 ist zum Gewindeschaft 4 verschwenkbar, bis die Tulpe 6 den Gewindeschaft 4, insbesondere dessen Schraubenschaft, kontaktiert und ein weiteres Verschwenken durch den Kontakt unterbunden ist. Mittels der Querausnehmungsachse 7.A und der Tulpenquerachse 6.A ist vorzugsweise eine Ebene gebildet, die parallel zur proximalen Stirnseite 6.pS liegt. Vorzugsweise liegt auch die distale Stirnseite 6.dS bis auf im Bereich der Abschrägung 11 parallel zu dieser Ebene. Insbesondere aus Fig.1d ist ersichtlich, dass die Tulpe 6 zur Abschrägung 11 hin stärker verschwenkt werden kann als in gegenüberliegender Richtung. Wenn die Tulpe 6 maximal zur Abschrägung 11 hin verschwenkt ist und den Gewindeschaft 4 im Bereich der Abschrägung 11 kontaktiert, bildet sich ein minimal möglicher Winkel zwischen der Mittelachse des Gewindeschafts 4 und der genannten Ebene aus. Die Tulpe 6 ist zum Gewindeschaft 4 hin maximal bezüglich der radialen Mittelachse 11.r verschwenkbar und in abweichender Richtung entsprechend weniger stark verschwenkbar.

Gemäß der zweiten Ausführungsform der medizinischen Schraube 1 aus Fig.2b weist die medizinische Schraube 1 zwei an oder von der distalen Stirnseite 6.dS der Tulpe 6 ausgebildete, den Schwenkwinkel α der Tulpe 6 bezüglich des Gewindeschafts 4 jeweils vergrößernde Aussparungen 12.1, 12.2 auf, deren, in Umfangsrichtung u gesehene, radiale Mittelachsen 12.1.r, 12.2.r in einer Tulpen-Radialrichtung auf einer Querausnehmungsachse 7.A und einer hierzu um 90° gedrehten Tulpenquerachse 6.A platziert sind.

Gemäß der Darstellung aus Fig.2b sind die radiale Mittelachse 12.1.r der ersten Aussparung 12.1 und die Querausnehmungsachse 7.A somit deckungsgleich. Gemäß der Darstellung aus Fig.2b sind weiterhin die radiale Mittelachse 12.2.r der zweiten Aussparung 12.2 und die Tulpenquerachse 6.A deckungsgleich. Dann ist ein vergrößertes Verschwenken der Tulpe 6 in zwei verschiedenen Richtungen zum Gewindeschaft 4 hin ermöglicht. Insbesondere ist ein Kontakt zwischen der jeweiligen kompletten Oberfläche der Aussparungen 12.1, 12.2 und dem Gewindeschaft 4, insbesondere dessen Schraubenschaft, erzeugbar, um eine gute Abstützung erreichen zu können.

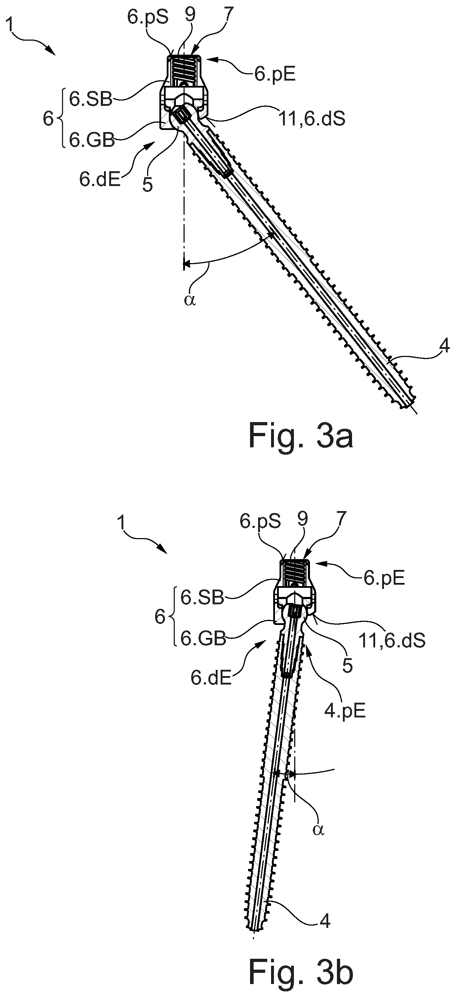

Gemäß der dritten Ausführungsform der medizinischen Schraube 1 aus Fig.3a und Fig.3b weist die medizinische Schraube 1 eine zumindest zweiteilige Tulpe 6 mit einem die Querausnehmung 7 aufweisenden Stab-Bereich 6.SB und mit einem den Kugelkopf 5 aufnehmenden Gelenk-Bereich 6.GB auf, welche vorzugsweise senkrecht zur Querausnehmungsachse 7.A verdrehbar zueinander sind, um eine in Umfangsrichtung u gesehene, radiale Mittelachse 11.r einer an oder von der distalen Stirnseite 6.dS der Tulpe 6 ausgebildeten, den Schwenkwinkel α der Tulpe 6 bezüglich des Gewindeschafts 4 vergrößernden Abschrägung 11 oder Aussparung relativ zur Querausnehmungsachse 7.A einzustellen.

Durch den in die Querausnehmung 7 eingesetzten Stabilisierungsstab 8 ist die Relativposition des Stab-Bereichs 6.SB zum Gelenk-Bereich 6.GB direkt oder indirekt fixierbar oder fixiert. Auch hier kann zur indirekten Fixierung ein Einsatz dazu dienen, das Bewegen des Stab-Bereichs 6.SB relativ zum Gelenk-Bereich 6.GB zu blockieren. Ein solcher Einsatz kann das Verdrehen des Stab-Bereichs 6.SB gegenüber dem Gelenk-Bereich 6.GB durch Form- und/oder Reibschluss unterbinden. Der Einsatz dient dann vorzugweise der Fixierung des Verdrehens des Stab-Bereichs 6.SB gegenüber dem Gelenk-Bereich 6.GB und auch des Verschwenkens der Tulpe 6 gegenüber dem Gewindeschaft 4.

Gemäß der ersten Ausführungsform der medizinischen Schraube 1 aus Fig.1a bis Fig.1d und Fig.2a ist die radiale Mittelachse 11.r der Abschrägung 11 um 40° bis 50°, bevorzugt um 43° bis 47°, insbesondere wie Fig.1b und Fig.2a zeigt um 45°, zur Querausnehmungsachse 7.A gedreht. Dann ist der mögliche Schwenkwinkel α der Tulpe 6 sowohl bezüglich der Querausnehmungsachse 7.A wie auch bezüglich der Tulpenquerachse 6.A vorzugsweise gleichartig vergrößert.

Der Neigungswinkel der Abschrägung 11 ist bezüglich der Mittelachse des Innengewindes 9 so gewählt, dass ein möglicher Schwenkwinkel α der Tulpe 6 in Richtung der Querausnehmungsachse 7.A und der 90° dazu gedrehten Tulpenquerachse 6.A jeweils Werte von 30° bis 46°, vorzugsweise von 35° bis 41°, insbesondere von 38°, aufweist.

Der mögliche Schwenkwinkel α der Tulpe 6 ist zwischen einer Mittestellung der Tulpe 6 und einer maximal zur Abschrägung 11 hin verschwenkten Tulpe 6 ausgebildet. In der Mittestellung der Tulpe 6 ist die Mittelachse des Gewindeschafts 4 senkrecht zur mittels der Querausnehmungsachse 7.A und der Tulpenquerachse 6.A aufgespannten Ebene ausgerichtet. In der Mittestellung der Tulpe 6 sind die Mittelachse des Gewindeschafts 4 und die Mittelachse des Innengewindes 9 vorzugsweise deckungsgleich oder liegen vorzugsweise zumindest parallel zueinander. Durch Realisierung derartig großer möglicher Schwenkwinkel α sind ein Großteil der sinnvollen Beckenverschraubungen wie auch sakroiliakalen Verschraubungen ermöglicht. Der maximal mögliche Schwenkwinkel α der Tulpe 6 zwischen der Mittestellung der Tulpe 6 und der maximal zur Abschrägung 11 hin verschwenkten Tulpe 6 ist zur radialen Mittelachse 11.r der Abschrägung 11 hin möglich, wobei dieser maximal mögliche Schwenkwinkel α dann entsprechend gegenüber den oben genannten möglichen Schwenkwinkeln α in Richtung der Querausnehmungsachse 7.A und der Tulpenquerachse 6.A vergrößert ist.

Gemäß der zweiten Ausführungsform der medizinischen Schraube 1 aus Fig.2b ist die Aussparungstiefe beider Aussparungen 12.1, 12.2 so gewählt, dass ein möglicher Schwenkwinkel α der Tulpe 6 in Richtung der Querausnehmungsachse 7.A und der 90° dazu gedrehten Tulpenquerachse 6.A jeweils Werte von 30° bis 46°, vorzugsweise von 35° bis 41°, insbesondere von 38°, aufweist. Auch mittels der zweiten Ausführungsform sind somit durch Realisierung derartig großer möglicher Schwenkwinkel α ein Großteil der sinnvollen Beckenverschraubungen wie auch sakroiliakalen Verschraubungen ermöglicht.

Ein möglicher Schwenkwinkel α der Tulpe 6 in den der Abschrägung 11 oder der Aussparungen 12.1, 12.2 abgewandten Richtungen weist vorzugsweise Werte von 5° bis 10°, vorzugsweise von 7° bis 8,2°, insbesondere von 7,6°, auf, wobei ein derartiger Schwenkwinkel α in Fig.3b mit einer entsprechend verschwenkten Tulpe 6 gezeigt ist.

Mittels der Aussparung 12.1, 12.2 oder Abschrägung 11 ist eine ebene Kontakt-Fläche zwischen der Tulpe 6 und dem Schraubenschaft gebildet. Die Oberfläche der Abschrägung 11 bildet somit eine Ebene. Demgegenüber ist auch denkbar, dass Oberfläche der Abschrägung eine Krümmung aufweist, solange sichergestellt ist, dass die radiale Mittelachse der Abschrägung definiert ist.

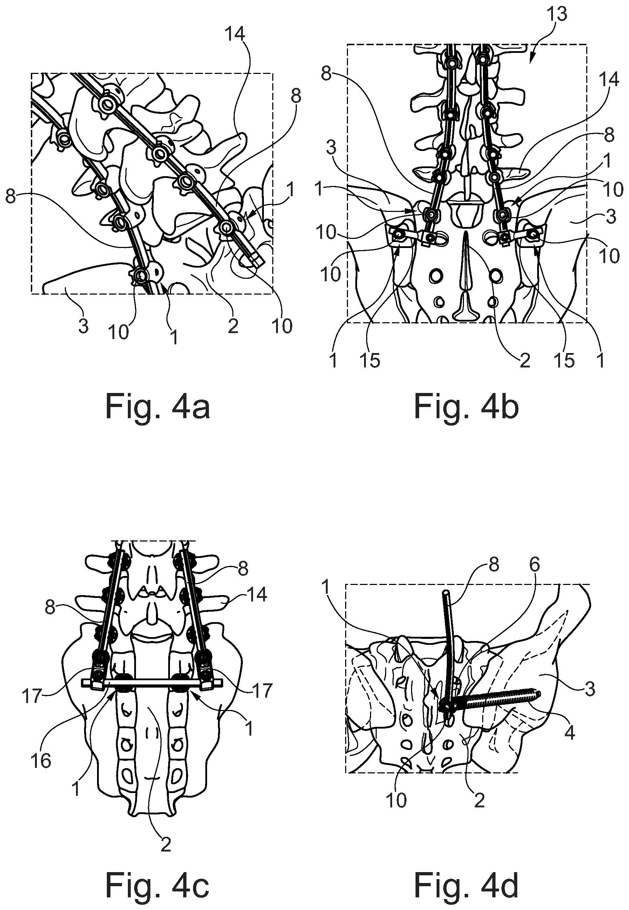

Fig.4a bis 4d zeigen verschiedene Anwendungsmöglichkeiten der medizinischen Schraube 1, wobei in Fig.4b eine medizinische Schraubenvorrichtung 13, ausgebildet und vorgesehen zum Einschrauben in das Sakrum 2 und das Ilium 3 eines Patienten sowie zum Stabilisieren der Verbindung zwischen dem Sakrum 2 und dem Ilium 3 gezeigt ist, welche wenigstens zwei im Vorhinein beschriebene medizinische Schrauben 1, einen Stabilisierungsstab 8 zur starren Kopplung des Sakrums 2 mit einem unmittelbar sich an diesen anschließenden Wirbelknochen 14, sowie einen Seitenversatz-Ausgleichsstab 15 zur starren Kopplung des Sakrums 2 mit dem Ilium 3 aufweist.

Die in Fig.4a bis 4d gezeigten medizinischen Schrauben 1 können jeweils nach einer der beschriebenen ersten, zweiten oder dritten Ausführungsform ausgeführt sein.

Fig.4a zeigt zwei jeweils von cranial nach caudal verlaufende Stabilisierungsstäbe 8 zur starren Kopplung des Sakrums 2 mit einem unmittelbar sich an diesen anschließenden Wirbelknochen 14. Ein jeder dieser zwei Stabilisierungsstäbe 8 ist mit einer medizinischen Schraube 1 verbunden, nämlich in deren Querausnehmung 7 aufgenommen und mit der zugehörigen Madenschraube 10 fixiert. Die medizinischen Schrauben 1 sind jeweils auf Höhe eines ersten und / oder zweiten Sakralwirbels in das Sakrum 2 eingeschraubt.

Fig.4b zeigt ebenso zwei jeweils von cranial nach caudal verlaufende Stabilisierungsstäbe 8 zur starren Kopplung des Sakrums 2 mit einem unmittelbar sich an diesen anschließenden Wirbelknochen 14. Ein jeder dieser zwei Stabilisierungsstäbe 8 ist mit einer medizinischen Schraube 1 verbunden, nämlich in deren Querausnehmung 7 aufgenommen und mit der zugehörigen Madenschraube 10 fixiert. Diese medizinischen Schrauben 1 sind jeweils auf Höhe des ersten und / oder zweiten Sakralwirbels in das Sakrum 2 eingeschraubt. Weiterhin ist jeweils ein medial nach lateral verlaufender Seitenversatz-Ausgleichsstab 15 mit einem jeden Stabilisierungsstab 8 verbunden, wobei jeweils eine weitere medizinischen Schraube 1 am jeweiligen vom Stabilisierungsstab 8 auskragenden Ende des Seitenversatz-Ausgleichsstabs 15 mit dem Seitenversatz-Ausgleichsstab 15 verbunden und in das Ilium 3 eingeschraubt ist. Dabei sind die Seitenversatz-Ausgleichsstäbe 15 in den Querausnehmungen 7 der zugehörigen medizinischen Schrauben 1 aufgenommen und mit den zugehörigen Madenschrauben 10 fixiert.

Fig.4c zeigt ebenso zwei jeweils von cranial nach caudal verlaufende Stabilisierungsstäbe 8 zur starren Kopplung des Sakrums 2 mit einem unmittelbar sich an diesen anschließenden Wirbelknochen 14. Die beiden Stabilisierungsstäbe 8 sind über einen weiteren Stab 16 miteinander verbunden, wobei die Verbindung zwischen dem Stab 16 und den zwei Stabilisierungsstäben 8 jeweils mittels eines Sakral-Connectors 17 realisiert ist. Der Stab 16 ist in den Querausnehmungen 7 zweier medizinischer Schrauben 1 aufgenommen, wobei diese beiden medizinischen Schrauben 1 jeweils in das Sakrum 2 eingeschraubt sind.

In Fig.4d ist ein einzelner Stabilisierungsstab 8 zur starren Kopplung des Sakrums 2 mit einem unmittelbar sich an diesen anschließenden Wirbelknochen gezeigt, welcher in der Querausnehmung 7 einer medizinischen Schraube 1 aufgenommen und mit der zugehörigen Madenschraube 10 fixiert ist. Diese medizinische Schraube 1 ist auf Höhe eines zweiten Sakralwirbels in das Sakrum 2 eingeschraubt. Diese medizinische Schraube 1 durchdringt das Sakrum 2 und ragt in das Ilium 3 hinein, so dass hier von einer sakroiliakalen Verschraubung gesprochen wird.

Insgesamt ist mit den medizinischen Schrauben 1 und mit der medizinischen Schraubenvorrichtung 13 eine gute Versorgung von Patienten bei entsprechenden Problemen im Bereich des Sakrums 2 und/oder des Iliums 3 ermöglicht, wobei baugleiche medizinische Schrauben 1 zu unterschiedlichen Zwecken eingesetzt werden können.

### Bezugszeichenliste

- 1: medizinische Schraube
- 2: Sakrum
- 3: Ilium
- 4: Gewindeschaft
- 4.pE: proximaler Endabschnitt des Gewindeschafts 4
- 5: Kugelkopf
- 6: Tulpe
- 6.dE: distaler axialer Endabschnitt der Tulpe 6
- 6.pE: proximaler axialer Endabschnitt der Tulpe 6
- 6.dS: distale Stirnseite der Tulpe 6
- 6.pS: proximale Stirnseite der Tulpe 6
- 6.A: Tulpenquerachse
- 6.SB: Stab-Bereich der Tulpe 6
- 6.GB: Gelenk-Bereich der Tulpe 6
- 7: Querausnehmung
- 7.A: Querausnehmungsachse
- 7.E: Einsatz (Insert)
- 8: Stabilisierungsstab
- 9: Innengewinde
- 10: Madenschraube
- 11: Abschrägung
- 11.r: radiale Mittelachse der Abschrägung
- 12.1: erste Aussparung
- 12.1.r: radiale Mittelachse der ersten Aussparung
- 12.2: zweite Aussparung
- 12.2.r: radiale Mittelachse der zweiten Aussparung
- 13: Medizinische Schraubenvorrichtung
- 14: Wirbelknochen
- 15: Seitenversatz-Ausgleichsstab
- 16: Stab
- 17: Sakral-Connector
- u: Umfangsrichtung
- α: Schwenkwinkel der Tulpe 6

## Patentansprüche

1. Medizinische Schraube (1), ausgebildet und vorgesehen zum wahlweisen Einschrauben in das Sakrum (2) und das Ilium (3) eines Patienten, mit
- einem Gewindeschaft (4), dessen proximaler Endabschnitt (4.pE) zu einem Kugelkopf (5) ausgebildet ist,
- einer hülsenförmigen Tulpe (6), die an ihrem distalen axialen Endabschnitt (6.dE) bezüglich des Gewindeschafts (4) schwenk- und drehbar am Kugelkopf (5) gehalten ist und die eine, an der proximalen Stirnseite (6.pS) der Tulpe (6) offene Querausnehmung (7) aufweist, die zur Queraufnahme eines Stabilisierungsstabs (8, 16) vorgesehen ist, wobei die Querausnehmung (7) im proximalen axialen Endabschnitt (6.pE) der Tulpe (6) mit einem Innengewinde (9) ausgebildet ist und
- einer Madenschraube (10), die in das Innengewinde (9) eingedreht oder eindrehbar ist sowie dafür vorgesehen ist, einen in die Querausnehmung (7) eingesetzten Stabilisierungsstab (8, 16) direkt oder indirekt gegen den Kugelkopf (5) zu drücken und die Tulpe (6) am Kugelkopf (5) in einer auswählbaren Relativausrichtung zu fixieren,
**gekennzeichnet durch**
eine, an oder von der distalen Stirnseite (6.dS) der Tulpe (6) ausgebildete, den Schwenkwinkel (α) der Tulpe (6) bezüglich des Gewindeschafts (4) vergrößernde Abschrägung (11), deren, in Umfangsrichtung (u) gesehene, radiale Mittelachse (11.r) in einer Tulpen-Radialrichtung zwischen einer Querausnehmungsachse (7.A) und einer hierzu um 90° gedrehten Tulpenquerachse (6.A) ausgerichtet ist.

2. Medizinische Schraube (1), ausgebildet und vorgesehen zum wahlweisen Einschrauben in das Sakrum (2) und das Ilium (3) eines Patienten, mit
- einem Gewindeschaft (4), dessen proximaler Endabschnitt (4.pE) zu einem Kugelkopf (5) ausgebildet ist,
- einer hülsenförmigen Tulpe (6), die an ihrem distalen axialen Endabschnitt (6.dE) bezüglich des Gewindeschafts (4) schwenk- und drehbar am Kugelkopf (5) gehalten ist und die eine, an der proximalen Stirnseite (6.pS) der Tulpe (6) offene Querausnehmung (7) aufweist, die zur Queraufnahme eines Stabilisierungsstabs (8, 16) vorgesehen ist, wobei die Querausnehmung (7) im proximalen axialen Endabschnitt (6.pE) der Tulpe (6) mit einem Innengewinde (9) ausgebildet ist und
- einer Madenschraube (10), die in das Innengewinde (9) eingedreht oder eindrehbar ist sowie dafür vorgesehen ist, einen in die Querausnehmung (7) eingesetzten Stabilisierungsstab (8, 16) direkt oder indirekt gegen den Kugelkopf (5) zu drücken und die Tulpe (6) am Kugelkopf (5) in einer auswählbaren Relativausrichtung zu fixieren,
**gekennzeichnet durch**
zwei an oder von der distalen Stirnseite (6.dS) der Tulpe (6) ausgebildete, den Schwenkwinkel (α) der Tulpe (6) bezüglich des Gewindeschafts (4) jeweils vergrößernde Aussparungen (12.1, 12.2), deren, in Umfangsrichtung (u) gesehene, radiale Mittelachsen (12.1.r, 12.2.r) in einer Tulpen-Radialrichtung auf einer Querausnehmungsachse (7.A) und einer hierzu um 90° gedrehten Tulpenquerachse (6.A) platziert sind.

3. Medizinische Schraube (1), ausgebildet und vorgesehen zum wahlweisen Einschrauben in das Sakrum (2) und das Ilium (3) eines Patienten, mit
- einem Gewindeschaft (4), dessen proximaler Endabschnitt (4.pE) zu einem Kugelkopf (5) ausgebildet ist,
- einer hülsenförmigen Tulpe (6), die an ihrem distalen axialen Endabschnitt (6.dE) bezüglich des Gewindeschafts (4) schwenk- und drehbar am Kugelkopf (5) gehalten ist und die eine, an der proximalen Stirnseite (6.pS) der Tulpe (6) offene Querausnehmung (7) aufweist, die zur Queraufnahme eines Stabilisierungsstabs (8, 16) vorgesehen ist, wobei die Querausnehmung (7) im proximalen axialen Endabschnitt (6.pE) der Tulpe (6) mit einem Innengewinde (9) ausgebildet ist und
- einer Madenschraube (10), die in das Innengewinde (9) eingedreht oder eindrehbar ist sowie dafür vorgesehen ist, einen in die Querausnehmung (7) eingesetzten Stabilisierungsstab (8, 16) direkt oder indirekt gegen den Kugelkopf (5) zu drücken und die Tulpe (6) am Kugelkopf (5) in einer auswählbaren Relativausrichtung zu fixieren,
**gekennzeichnet durch**
eine zumindest zweiteilige Tulpe (6) mit einem die Querausnehmung (7) aufweisenden Stab-Bereich (6.SB) und mit einem den Kugelkopf (5) aufnehmenden Gelenk-Bereich (6.GB), welche vorzugsweise senkrecht zur Querausnehmungsachse (7.A) verdrehbar zueinander sind, um eine in Umfangsrichtung (u) gesehene, radiale Mittelachse (11.r), einer an oder von der distalen Stirnseite (6.dS) der Tulpe (6) ausgebildeten, den Schwenkwinkel (α) der Tulpe (6) bezüglich des Gewindeschafts (4) vergrößernden Abschrägung (11) oder Aussparung relativ zur Querausnehmungsachse (7.A) einzustellen.

4. Medizinische Schraube (1) nach Anspruch 1 **dadurch gekennzeichnet, dass** die radiale Mittelachse (11.r) der Abschrägung (11) um 40° bis 50°, bevorzugt um 43° bis 47°, insbesondere um 45°, zur Querausnehmungsachse (7.A) gedreht ist.

5. Medizinische Schraube (1) nach Anspruch 1, **dadurch gekennzeichnet, dass** der Neigungswinkel der Abschrägung (11) bezüglich der Mittelachse des Innengewindes so gewählt ist, dass ein möglicher Schwenkwinkel (α) der Tulpe (6) in Richtung der Querausnehmungsachse (7.A) und der 90° dazu gedrehten Tulpenquerachse (6.A) jeweils Werte von 30° bis 46°, vorzugsweise von 35° bis 41°, insbesondere von 38°, aufweist.

6. Medizinische Schraube (1) nach Anspruch 2, **dadurch gekennzeichnet, dass** die Aussparungstiefe beider Aussparungen (12.1, 12.2) so gewählt ist, dass ein möglicher Schwenkwinkel (α) der Tulpe (6) in Richtung der Querausnehmungsachse (7.A) und der 90° dazu gedrehten Tulpenquerachse (6.A) jeweils Werte von 30° bis 46°, vorzugsweise von 35° bis 41°, insbesondere von 38°, aufweist.

7. Medizinische Schraube (1) nach einem der vorangehenden Ansprüche 1 bis 6, **dadurch gekennzeichnet, dass** mittels der Aussparung (12.1, 12.2) oder Abschrägung (11) eine ebene Kontakt-Fläche zwischen der Tulpe (6) und dem Schraubenschaft gebildet ist.

8. Medizinische Schraubenvorrichtung (13), ausgebildet und vorgesehen zum Einschrauben in das Sakrum (2) und das Ilium (3) eines Patienten sowie zum Stabilisieren der Verbindung zwischen dem Sakrum (2) und dem Ilium (3), **gekennzeichnet durch** wenigstens zwei medizinische Schrauben (1) nach einem der Ansprüche 1 bis 7, einem Stabilisierungsstab (8) zur starren Kopplung des Sakrums (2) mit einem unmittelbar sich an diesen anschließenden Wirbelknochen (14), sowie einem Seitenversatz-Ausgleichsstab (15) zur starren Kopplung des Sakrums (2) mit dem Ilium (3).
